# EUROPÄISCHE PATENTSCHRIFT

(11) **EP 0 894 852 B1**
(45) Veröffentlichungstag und Bekanntmachung des Hinweises auf die Patenterteilung: **28.01.2004**
(21) Anmeldenummer: 98113394.5
(22) Anmeldetag: 17.07.1998
(51) Int. Cl.: C12M 1/40

(54) **Verfahren und Vorrichtung zur Durchführung biochemischer Reaktionen**
Process and apparatus for performing biochemical reactions
Procédé et appareil pour la réalisation de réactions biochimiques

(30) Priorität: 31.07.1997 DE 19732935
(43) Veröffentlichungstag der Anmeldung: 03.02.1999
(73) Patentinhaber: Roche Diagnostics GmbH, 68298 Mannheim (DE)
(72) Erfinder: Bauer, Hermann, Prof. Dr., 91207 Lauf (DE); Menzler, Horst, 82347 Bernried (DE); Kleinhammer, Gerd, Dr., 82327 Tutzing (DE); Schels, Hans, Dr., 80687 München (DE); Bendzko, Peter, Dr., 12623 Berlin (DE); Fink, Ute, 90489 Nürnberg (DE); Maciej, Bernd, 90453 Nürnberg (DE)
(74) Vertreter: Pfeifer, Hans-Peter, Dr.

(56) Entgegenhaltungen:
- WO-A-84/01959
- WO-A-86/02379
- WO-A-94/18341
- US-A- 5 135 853
- US-A- 5 478 730

## Beschreibung

Die Erfindung betrifft ein Verfahren und eine Vorrichtung zur Durchführung biochemischer Reaktionen, insbesondere für die zellfreie Biosynthese von Proteinen oder anderen Polypeptiden.

In der Biotechnologie werden biochemische Reaktionen üblicherweise in Bioreaktoren durchgeführt. Beispiele solcher biochemischen Reaktionen sind enzymatische Reaktionen, bei denen ein Substrat durch die katalytische Wirkung eines Enzyms von einem Ausgangszustand in einen Endzustand umgeformt wird, sowie die Biosynthese von Naturstoffen, beispielsweise Peptidhormonen, Antibiotika und - in neuerer Zeit von besonderer Bedeutung - Humanproteinen.

Die dabei insgesamt biochemisch wirksamen Komponenten kann man allgemein als "produzierendes System" bezeichnen. Das produzierende System enthält fast immer Komponenten, die sehr teuer sind, sich jedoch während der Reaktion nicht oder nur in geringem Umfang verbrauchen. Sie werden deshalb innerhalb des Bioreaktors in einem Reaktionsraum immobilisiert. Die Erfindung richtet sich auf Verfahren, bei denen der Reaktionsraum innerhalb des Bioreaktors durch eine semipermeable Membran von einem Versorgungsraum getrennt ist.

Ein Verfahren zur zellfreien Biosynthese von Polypeptiden ist beispielsweise in der US-Patentschrift 5,478,730 beschrieben. Das produzierende System enthält dabei eine Quelle von Boten-RNA (mRNA), die das Polypeptid codiert. In dem zellfreien Synthesesystem sind weiterhin Ribosomen, tRNA, Aminosäuren, ATP- und GTP enthalten. Die Translation der mRNA mit Hilfe der tRNA führt zur Produktion des Polypeptids, wobei zugleich Produkte mit niedrigerem Molekulargewicht entstehen. Der Reaktionsraum mit dem produzierenden System ist durch eine semipermeable Barriere von einem Versorgungsraum getrennt. Der Versorgungsraum enthält eine als Versorungsmedium für das produzierende System wirkende Flüssigkeit, welche ATP, GTP und Aminosäuren enthält. Diese Komponenten werden dem produzierenden System zugeführt, um den Verbrauch während der Bio-Reaktion zu ersetzen. Sie können durch die semi-permeable Barriere diffundieren, weil ihr Molekulargewicht unterhalb von deren Durchlaßgrenze liegt. Zugleich diffundieren Produkte der biochemischen Reaktion und andere Substanzen, deren Molekulargewicht unterhalb der Durchlaßgrenze der Barriere liegt, aus dem Reaktionsraum in den Versorgungsraum.

Als semipermeable Barriere werden in der US-Patentschrift 5,478,730 semipermeable Membranen, insbesondere in der Form von Membran-Hohlfasern (nachfolgend "Kapillarmembranen") empfohlen. Die Durchlaßgrenze der semipermeablen Membran wird so gewählt, daß durch sie hindurch die niedermolekularen Reaktionskomponenten, die zur Aufrechterhaltung der biochemischen Reaktion notwendig sind, zugeführt und relativ niedermolekulare Produkte aus dem Reaktionsraum entfernt werden können. Dagegen werden die mRNA und andere besonders hochwertige hochmolekulare Bestandteile des produzierenden Systems in dem Reaktionsraum festgehalten. Bevorzugt liegt die Durchlaßgrenze unter 100.000 Dalton, vorzugsweise unter 50.000 Dalton. Sie sollte aber über 1.000 bis 5.000 Dalton liegen.

Die zitierte US-Patentschrift enthält umfangreiche weitere Erläuterungen über geeignete Zusammensetzungen des produzierenden Systems und des Versorgungsmediums sowie über die Durchführung des Biosynthese-Verfahrens. Insoweit bezieht sich die vorliegende Erfindung auf den vorbekannten Stand der Technik, wie er insbesondere der US-Patentschrift 5,478,730 und den darin zitierten Literaturstellen zu entnehmen ist.

WO-A-94/18341 offenbart einen Bioreaktor zur zellfreien Polypeptid-Biosynthese mit zwei getrennten Versorgungsräumen, die jeweils über eine semipermeable Membran mit dem Reaktionsraum in Verbindung stehen und über eine Verbindungsleitung miteinander verbunden sind. Das produzierende System und die Versorgungsflüssigkeit werden gemischt und zusammen in dem Bioreaktor eingeführt. US-A-5,135,853 beschreibt einen Bioreaktor für biochemische Reaktionen mittels eines Biokatalysators.

Soweit die Erfindung für andere biochemische Reaktionen als Polypeptidsynthesen verwendet wird, wird ein dem jeweiligen Anwendungszweck angepaßtes produzierendes System eingesetzt. Auch hierzu können nähere Angaben der einschlägigen Literatur entnommen werden. Ein produzierendes System kann grundsätzlich alles sein, was in der Lage ist, die Umsetzung bzw. Synthese gewünschter Verbindungen auf biochemischem Wege zu ermöglichen oder zu beschleunigen. Neben dem genannten System kommen insbesondere Enzyme und Enzymkomplexe in Verbindung mit den bei derartigen Verfahren gebräuchlichen Hilfssubstanzen in Betracht.

Da die für die Biosynthese von Polypeptiden erforderliche mRNA meist nur in sehr geringen Mengen zu vertretbaren Kosten zur Verfügung steht und auch die hergestellten Produktmengen sehr klein sind, werden solche Reaktionen vielfach in sehr kleinen Bioreaktoren durchgeführt. Die Erfindung richtet sich insbesondere auf Mikro-Membranreaktoren, die üblicherweise ein Reaktionsraumvolumen von etwa 1 ml (jedenfalls von weniger als 10 ml) haben. Da es sich dabei um zur einmaligen Verwendung vorgesehene Einwegprodukte handelt, kommt es besonders darauf an, daß solche Mikro-Membranreaktoren zu günstigen Kosten hergestellt werden können.

Der Erfindung liegt die Aufgabe zugrunde, ein Verfahren und eine Vorrichtung zur Verfügung zu stellen, mit denen die Durchführung biochemischer Reaktionen mit einer hohen Effektivität und zugleich kostengünstig möglich ist.

Die Aufgabe wird gelöst durch ein Verfahren und eine Vorrichtung gemäß den Ansprüchen 1 bis 20.

Eine erfindungsgemäße Vorrichtung kann auch als Bioreaktor-System bezeichnet werden, wobei die Pumpe und die Verbindungsleitung entweder von dem Bioreaktor-Modul des Systems getrennte, funktionell an diesen angepaßte Systembestandteile sind, oder in das Gehäuse des Bioreaktors integriert sein können.

Während bei vorbekannten Bioreaktoren üblicherweise innerhalb des Reaktorgehäuses ein einziger Versorgungsraum vorhanden ist, besteht bei der Erfindung der Gesamt-Versorgungsraum innerhalb des Bioreaktors aus zwei Teilräumen, die durch eine eine Pumpe enthaltende Verbindungsleitung in Fluidverbindung stehen, im übrigen aber voneinander getrennt sind. Über die Verbindungsleitung kann mittels der Pumpe Flüssigkeit von dem einen Teilraum in den anderen Teilraum gepumpt werden. Jeder der Teilräume steht über eine Versorgungsmembran mit dem Reaktionsraum in Verbindung. Bei Betätigung der Pumpe entsteht in dem stromaufwärts gelegenen Teilraum ein Überdruck. Dadurch wird eine Strömung der Flüssigkeit durch die Versorgungsmembran in den Reaktionsraum verursacht. Der daraus resultierende Überdruck in dem Reaktionsraum führt dazu, daß simultan ein Flüssigkeitsstrom aus dem Reaktionsraum in den zweiten Teilraum stattfindet. Von dort wird Flüssigkeit über die Verbindungsleitung und die Pumpe abgepumpt, so daß ein Kreislauf entsteht. Sofern gemäß einer bevorzugten Ausführungsform das Flüssigkeitsvolumen in den verschiedenen Teilräumen des Kreislauf-Systems (Reaktionsraum, beide Teilräume des Versorgungsraums) unverändert bleibt, ist zu jedem Zeitpunkt der Volumenstrom der Flüssigkeit in den Reaktionsraum hinein und aus dem Reaktionsraum heraus gleich groß. Es wird dem Reaktionsraum also je Zeiteinheit ebensoviel Flüssigkeit aus dem ersten Teilraum zugeführt wie aus dem zweiten Teilraum abgeführt wird.

Bevorzugt sind innerhalb eines Bioreaktors ein einziger Reaktionsraum und genau zwei Teilräume des Versorgungsraums vorhanden. Grundsätzlich ist es jedoch auch möglich, beide Räume, insbesondere den Versorgungsraum, in mehrere Teilräume aufzuteilen. Beispielsweise könnte der Versorgungsraum in vier Teilräume aufgeteilt sein, wobei die Flüssigkeit im wesentlichen paarweise von dem ersten und zweiten Teilraum in den dritten und vierten Teilraum strömt.

Durch die Erfindung wird eine sehr gute Versorgung und Entsorgung des produzierenden Systems gewährleistet. Daraus resultiert eine hohe Produktionsrate. Das erforderliche Bioreaktor-System ist klein, verhältnismäßig einfach aufgebaut und kann deswegen zu günstigen Kosten hergestellt werden.

Selbstverständlich kann der Umpumpvorgang während der biochemischen Reaktion unterbrochen werden, wobei jedoch ein zumindest weitgehend kontinuierlicher Betrieb der Pumpe (während mindestens 80% der Reaktionszeit) bevorzugt ist.

Gemäß einer bevorzugten Ausführungsform wird während der biochemischen Reaktion die Pumprichtung und damit die Strömungsrichtung der Flüssigkeit umgekehrt, wobei besonders bevorzugt die Umkehrung der Pumprichtung während einer biochemischen Reaktion mehrfach erfolgt und gemäß einer weiteren bevorzugten Ausführungsform das jeweils in eine Richtung (bis zur nächsten Umkehrung der Pumprichtung) gepumpte Volumen mindestens so groß wie das Volumen des Reaktionsraums ist.

Eine solche Betriebsweise, die auch als "Modulation" der Strömung bezeichnet werden kann, hat den Vorteil, daß die Bildung einer sogenannten Sekundärfilterschicht auf der Oberfläche der semipermeablen Membranen vermieden wird. Eine Sekundärfilterschicht entsteht dadurch, daß sich während des Durchströmens der Membran in eine Richtung vor der Membranwand ein Konzentrationsgradient der durch die Membran zurückgehaltenen Stoffe ausbildet. Dadurch werden die Filtrationseigenschaften der Membran beeinträchtigt. Die Vermeidung einer Sekundärfilterschicht durch Modulation der Strömungsrichtung ist aus der DE 3914956 A1 bekannt.

Dieser Druckschrift ist auch zu entnehmen, daß es vorteilhaft ist, eine asymmetrische semipermeable Membran derartig einzusetzen, daß die eigentliche Filtrationsschicht zu dem Reaktionsraum orientiert ist. Auch bei der vorliegenden Erfindung wird bevorzugt eine solche asymmetrische Membran gleicher Orientierung zur Trennung des Reaktionsraums und des Versorgungsraums eingesetzt.

Die DE 3914956 A1 enthält umfangreiche Erläuterungen hinsichtlich des Modulationsverfahrens sowie hinsichtlich des Aufbaus und der Orientierung geeigneter Membranen, die auch auf die vorliegende Erfindung unter Berücksichtigung von deren Besonderheiten anwendbar sind. Insoweit wird der Inhalt der genannten Druckschrift durch Bezugnahme zum Inhalt der vorliegenden Erfindungsbeschreibung gemacht. Ein besonderer Aufbau einer asymmetrischen Membran ist Gegenstand der WO 94/20603.

Die Erfindung wird im folgenden anhand von in den Figuren dargestellten Ausführungsbeispielen näher erläutert. Es ze igen :
- Fig. 1: eine stark schematisierte Querschnittsdarstellung einer erfindungsgemäßen Vorrichtung;
- Fig. 2: eine schematisierte Querschnittsdarstellung einer alternativen Ausführungsform einer erfindungsgemäßen Vorrichtung;
- Fig. 3: eine Explosionsdarstellung von Teilen eines für die Erfindung geeigneten Bioreaktor-Moduls;
- Fig. 4: eine graphische Darstellung der Antwortfunktion beim Betrieb eines Bioreaktor-Systems entsprechend der Erfindung,
- Fig. 5: einen Ausschnitt aus einer Querschnittsdarstellung analog Fig. 1 von einer alternativen Ausführungsform,
- Fig. 6: eine Explosionsdarstellung analog Fig. 3 von einer alternativen Ausführungsform,
- Fig. 7: eine Aufsicht auf eine weitere alternative Ausführungsform eines Bioreaktor-Moduls,
- Fig. 8: einen Detailschnitt entlang der Linie VIII in Fig. 7,
- Fig. 9: einen Detailschnitt entlang der Linie IX von Fig. 7.

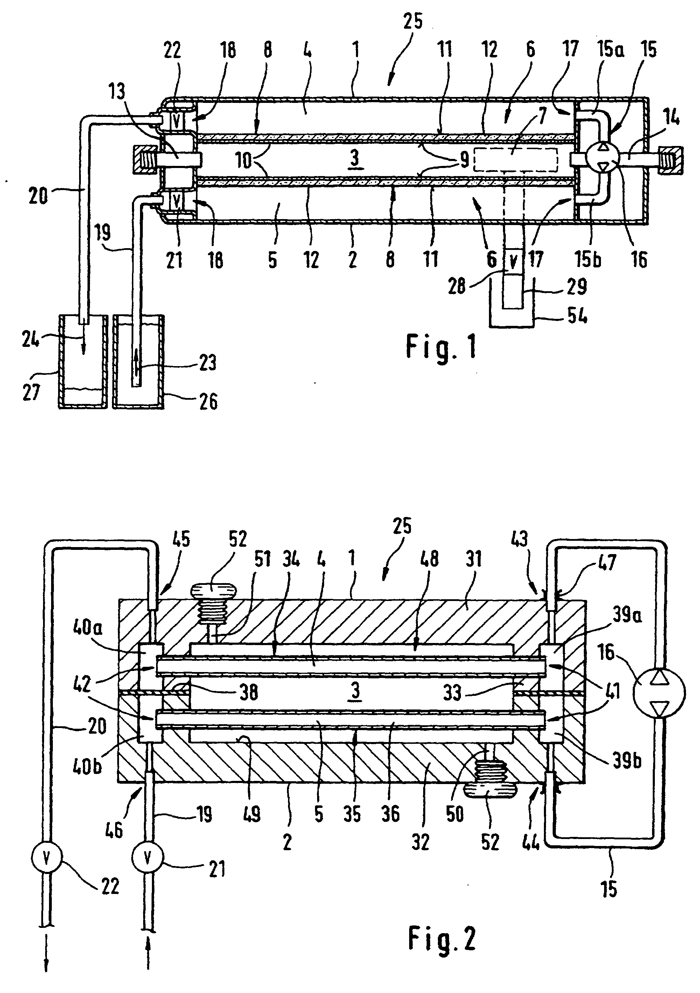

Der in Fig. 1 dargestellte Bioreaktor 1 enthält innerhalb eines Gehäuses 2 einen Reaktionsraum 3 und zwei Teilräume 4, 5 eines Versorgungsraums 6. Die beiden Teilräume 4, 5 stehen jeweils über eine Versorgungsmembran 8 mit dem Reaktionsraum 3 in Verbindung. Die Versorgungsmembranen 8 weisen jeweils eine einer ersten Oberfläche 9 zugewandte Filtrationsschicht 10 und eine einer zweiten Oberfläche 11 zugewandte Tragschicht 12 auf. Sie sind so orientiert, daß die erste Oberfläche 9 (d.h. die Filtrationsschicht) dem Reaktionsraum 3 zugewandt ist.

Die beiden Teilräume 4, 5 des Versorgungsraums 6 sind über eine Verbindungsleitung 15 verbunden, in der eine Pumpe 16 angeordnet ist, welche Flüssigkeit in beide Flußrichtungen durch die Leitung 15 pumpen kann. Die Teilräume 4, 5 des Versorgungsraums 6 sind jeweils geschlossen, mit Ausnahme der Durchlässigkeit der Membranen 8 und mit Ausnahme von jeweils zwei Anschlußöffnungen, von denen jeweils eine erste Öffnung 17 eine Flüssigkeitsverbindung zu der Verbindungsleitung 15 und eine zweite Öffnung 18 eine Flüssigkeitsverbindung zu Veroder Entsorgungsleitungen 19, 20 herstellt, die jeweils durch Ventile 21, 22 verschließbar sind. Die Anschlußöffnungen 17, 18 sind vorzugsweise - wie dargestellt - an gegenüberliegenden Enden der insgesamt länglichen Teilräume 4, 5 vorgesehen.

Bei der dargestellten Ausführungsform befindet sich die Verbindungsleitung 15 und die Pumpe 16 innerhalb des Gehäuses 2 des Bioreaktors 1. Auch die Ventile 21, 22 sind bevorzugt - wie dargestellt - in das Gehäuse 2 des Bioreaktors 1 integriert.

Eine biochemische Reaktion wird in dem in Fig. 1 dargestellten, insgesamt mit 25 bezeichneten Bioreaktorsystem folgendermaßen durchgeführt.

Der Reaktionsraum 3 wird über Anschlußöffnungen 13 und 14 mit einer das produzierende System enthaltenden Flüssigkeit gefüllt. Die Versorgungsleitung 19 wird an einen Behälter 26 mit Versorgungsmedium angeschlossen. Die Entsorgungsleitung mündet in einen zunächst leeren Behälter 27 für das Eluat. Die Ventile 21 und 22 werden geöffnet und mittels der Pumpe 16 oder einer zusätzlichen in der Versorgungsleitung 19 vorgesehenen Pumpe wird Flüssigkeit zunächst in den zweiten Teilraum 5 und dann in den ersten Teilraum 4 des Versorgungsraums 6 in Richtung der Pfeile 23, 24 transportiert, bis beide Teilräume sowie das Volumen der Leitungen 19 und 20 bis zu den Ventilen 21, 22 vollständig gefüllt sind.

Danach werden die Ventile 21, 22 geschlossen. Wenn die Pumpe 16 bei geschlossenen Ventilen 21, 22 in Betrieb gesetzt wird, entsteht ein Überdruck in dem entsprechenden Teilraum, also beispielsweise in dem Teilraum 4, wenn die Pumpe 16 in den in Fig. 1 oberen Abschnitt 15a der Verbindungsleitung 15 hineinpumpt. Aufgrund des Drucks strömt Flüssigkeit aus dem Teilraum 4 in den Reaktionsraum 3. Auch dort entsteht ein Überdruck, durch den die Flüssigkeit weiter strömt in den zweiten Teilraum 5. Von dort strömt sie - wiederum unter Einwirkung der Pumpe - in den unteren Abschnitt 15b der Verbindungsleitung 16 und von dort im Kreislauf in den ersten Teilraum 4.

Nachdem auf diese Weise ein Volumen, das mindestens so groß ist wie das Volumen des Reaktionsraums 3 umgepumpt worden ist, wird die Wirkungsrichtung der Pumpe 16 umgedreht, so daß eine umgekehrte Strömung einsetzt. Dieser Vorgang kann während der gesamten Reaktionszeit mehrfach wiederholt werden.

Nach Ablauf einer gewünschten Reaktionszeit werden die Ventile 21 und 22 geöffnet und erneut ein Pumpvorgang im Sinne der Pfeile 23, 24 eingeleitet, um das Versorgungsmedium, welches nun einen erheblichen Anteil des gewünschten Produkts enthält, in den Eluatbehälter 27 zu transportieren. Zugleich wird frisches Versorgungsmedium in die Teilräume 4, 5 des Versorgungsraums 6 geleitet. Nach vollständigem Austausch des Inhalts des Versorgungsraums kann eine neue biochemische Reaktion begonnen werden.

Bei der vorstehend beschriebenen Verfahrensweise wird das gewünschte Produkt aus dem Eluat des Versorgungsmediums gewonnen. Dies setzt voraus, daß die Poren der Versorgungsmembranen 8 groß genug sind, daß das in dem Reaktionsraum 3 erzeugte Produkt während des beschriebenen Umpumpvorganges in den Versorgungsraum 6 übertritt. Dies setzt (abhängig von dem Molekulargewicht der gewünschten Produkte) vielfach eine relativ grobporige Membran mit einer Durchlaßgrenze von etwa 50.000 Dalton voraus. Eine solch hohe Durchlaßgrenze führt jedoch dazu, daß nicht nur das Produkt und relativ niedermolekulare Bestandteile der Versorgungsflüssigkeit (ATP, GTP und Aminosäuren) aus dem Reaktionsraum 3 herausgepumpt werden, sondern auch höhermolekulare und besonders wertvolle Bestandteile des produzierenden Systems, die zurückgehalten werden sollten.

Deshalb ist es bei der Produktion relativ hochmolekularer Produkte vorteilhafter, das Produkt unmittelbar aus dem Reaktionsraum zu gewinnen ("ernten"). Zu diesem Zweck kann gemäß einer vorteilhaften Ausführungsform eine zusätzliche Erntemembran 7 in Kontakt zu dem Reaktionsraum so angeordnet sein, daß zum Ernten des von dem produzierenden Systems erzeugten Produkts Flüssigkeit aus dem Reaktionsraum 3 über die Erntemembran abgeleitet werden kann. In Figur 1 ist die Erntemembran 7 in einer den Reaktionsraum 3 begrenzenden Wand eingelassen und steht über eine mit einem Ventil 28 verschließbare Leitung 29 mit einem Behälter 54 zur Aufnahme der durch die Erntemembran 7 aus dem Reaktionsraum 3 abgeleiteten Flüssigkeit in Verbindung. Die Durchlaßgrenze der Erntemembran 7 ist größer als die der Versorgungsmembranen 8. Bei der Ernte aus dem Reaktionsraum sind zwei unterschiedliche Betriebsweisen möglich.

Einerseits kann während des Betriebes der Pumpe 16 das Ventil 28 geöffnet werden. Durch den in dem Reaktionsraum 3 herrschenden Überdruck wird in diesem Fall ständig Flüssigkeit aus dem Reaktionsraum 3 über die Erntemembran 7 und durch die Leitung 29 abgeführt. Die Menge läßt sich durch die Stellung des Ventils 28 regeln.

Im Regelfall bevorzugt ist jedoch eine Betriebsweise, die man als Batch-Ernteverfahren bezeichnen kann. Dabei wird erst am Ende des Biosynthese-Vorgangs, wenn in dem Reaktionsraum 3 eine erhöhte Produktkonzentration erreicht ist, über die Erntemembran 7 geerntet. Dies geschieht bevorzugt in der Weise, daß das Ventil 22 geschlossen wird und mittels der Pumpe 16 die Versorgungsflüssigkeit weiter in den Reaktionsraum gedrückt wird, so daß die in dem Reaktionsraum 3 enthaltene Flüssigkeit durch die Erntemembran abfließt. Alternativ kann eine der Anschlußöffnungen 13 oder 14 verwendet werden, um durch Einleiten einer geeigneten Flüssigkeit in den Reaktionsraum die darin enthaltene Flüssigkeit durch die Erntemembran 7 zu verdrängen.

Die Ernte über eine zusätzliche Erntemembran hat den Vorteil, daß die Versorgungsmembranen 8 feiner (beispielsweise mit einer Durchlaßgrenze von weniger als 30.000 Dalton) ausgebildet sein können. Dadurch werden Verluste an wertvollen Substanzen aus dem Reaktionsraum 3 minimiert. Andererseits kann die Erntemembran 7 eine relativ hohe Durchlaßgrenze (mehr als 70.000 Dalton) haben. Die dadurch verursachten Verluste an wertvollen Bestandteilen bei der Ernte sind insbesondere im Falle des Batch-Ernteverfahrens relativ gering, weil am Ende der Biosynthese eine relativ hohe Produktkonzentration erreicht ist.

Bei dem Bioreaktorsystem 25 von Fig. 1 ist die Verbindungsleitung 15 und die Pumpe 16 im Gehäuse des Bioreaktors 2 integriert. Als zusätzliche Elemente des Systems sind nur noch die Leitungen 19, 20 und die Ventile 21, 22 erforderlich. Die Pumpe 16 kann grundsätzliche eine elektrische Mikropumpe sein. Bei der in Fig. 1 dargestellten Ausführungsform mit integrierter Pumpe ist es jedoch möglicherweise vorteilhaft, einen einfacheren Pumptyp zu verwenden. In Frage kommt beispielsweise eine Kolbenpumpe mit einem durch Federspannung betätigten Kolben, wobei das Spannen der Feder manuell erfolgen kann. Die Kolbenpumpe sollte in diesem Fall einen Hubraum haben, der mindestens dem Volumen des Reaktionsraums 3 entspricht. Zum Füllen und Entleeren des Versorgungsraums 6 wird in diesem Fall zweckmäßigerweise eine in der Figur nicht dargestellte, in die Leitung 19 integrierte Elektropumpe eingesetzt.

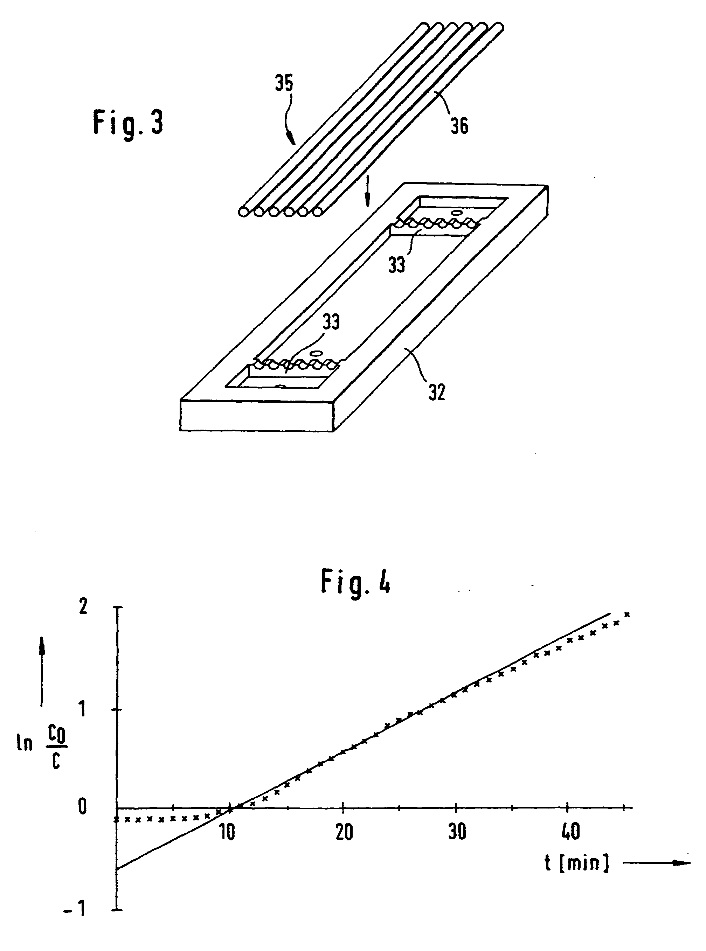

Bei der in den Figuren 2 und 3 dargestellten Ausführungsform sind entsprechende Teile mit den gleichen Bezugsziffern wie bei Fig. 1 bezeichnet, auch wenn sie in konstruktiven Einzelheiten anders gestaltet sind. Während in Fig. 1 eine Flachmembran (beispielsweise eine Membranfilterschicht 10 auf einem Träger aus Sintermaterial 12) verwendet wird, ist der in Fig. 2 dargestellte Bioreaktor 1 mit Membranhohlfasern ausgestattet, die nachfolgend als "Kapillaren" bezeichnet werden.

Das Gehäuse 2 des Bioreaktors 1 besteht bei dieser Ausführungsform aus zwei Hälften 31 und 32, wobei jede der Hälften Abschlußwände 33 aufweist, in die ein Satz 34 bzw. 35 von in einer gemeinsamen Ebene parallel verlaufenden Kapillaren 36 dichtend eingelassen ist. Die beiden Hälften 31, 32 werden mit nicht dargestellten Mitteln (beispielsweise Klammern oder Schrauben) zusammengedrückt, wobei sich zwischen ihnen eine flache Dichtung 38 befindet. Die beiden Hälften können auch durch Kleben oder Schweißen dicht und fest miteinander verbunden sein.

Durch die dargestellte Konstruktion wird jeweils an beiden Enden jedes Satzes 34, 35 von Kapillaren 36 ein Sammelraum 39a, 39b, 40a, 40b ausgebildet, der mit einem Ende der Innenräume der Kapillaren eines Satzes und mit einer Anschlußöffnung 43, 44, 45, 46 in Verbindung steht, jedoch im übrigen abgeschlossen ist. Dabei steht jeweils ein erstes Ende 41 der Kapillaren 36 über den entsprechenden Sammelraum 39a bzw. 39b mit einer Anschlußöffnung 43 bzw. 44 für die Verbindungsleitung und ein zweites Ende 42 über einen ihm zugeordneten Sammelraum 40a bzw. 40b mit einer Anschlußöffnung 45 bzw. 46 für eine Versorgungsleitung 19 bzw. eine Entsorgungsleitung 20 in Verbindung. Die Sammelräume 39a, 39b, 40a, 40b bilden somit in Verbindung mit den jeweils zugehörigen Anschlußöffnungen 43, 44; 45, 46 jeweils einen gemeinsamen Leitungsanschluß für einen Satz von Kapillaren 34 bzw. 35. Die Innenräume der Kapillaren des Kapillarensatzes 34 bilden zusammen mit den Sammelräumen 39a, 40a den ersten Teilraum 4 des Versorgungsraums 6, während die Innenräume des Kapillarensatzes 35 gemeinsam mit den Sammelräumen 39b, 40b den zweiten Teilraum 5 des Versorgungsraums 6 bildet.

Der Reaktionsraum 3 wird bei dieser Ausführungsform durch den Außenraum 48 zwischen den Kapillaren 36 und der Wand 49 des Gehäuses 2 gebildet. Er kann über eine Einlaßöffnung 50 und eine diametral gegenüber angeordnete Entlüftungsöffnung 51 blasenfrei mit einer das produzierende System enthaltenden Flüssigkeit gefüllt werden. Während der biochemischen Reaktion sind die Öffnungen 50, 51 jeweils, beispielsweise mit einem Stöpsel 52, verschlossen. Die Kapillaren 36 der beiden Kapillarensätze 34, 35 verlaufen im wesentlichen parallel zueinander durch den Reaktionsraum 3.

Die Verbindungsleitung 15, in die wiederum eine Pumpe 16 integriert ist, verläuft bei der hier dargestellten Ausführungsform außerhalb des Bioreaktors 1 und ist mittels lediglich symbolisch dargestellter Kupplungen 47 angeschlossen. Das Bioreaktorsystem 25 wird hier insgesamt durch den Bioreaktor 1, die Leitungen 15, 19, 20, die Ventile 21, 22 und die Pumpe 16 gebildet.

Die Verwendung von Kapillaren statt flacher Membranen ist bevorzugt, weil sich auf einfache Weise und kostengünstig eine große Membranfläche realisieren läßt. Ebenso wie bei Fig. 1 sind die Membranen der Kapillaren 36 bevorzugt asymmetrisch mit dem Reaktionsraum 3 zugewandter Filterschicht ausgebildet (vgl. DE 3114956 A1).

Die Durchführung einer biochemischen Reaktion mit dem Bioreaktorsystem gemäß Fig. 2 und 3 erfolgt analog wie bei Fig. 1.

Fig. 4 zeigt die Antwortfunktion eines Bioreaktors gemäß den Figuren 2 und 3. Dabei wird die Änderung der Konzentration einer zu Beginn des Versuchs in dem Reaktionsraum 3 enthaltenen, leicht nachweisbaren niedermolekularen Substanz während des Betriebs des Bioreaktors über die Zeit verfolgt. Diese "Modellreaktion" wurde nach 10 Minuten gestartet und für etwa weitere 35 Minuten fortgeführt, wobei die Pumpe 16 ständig in Betrieb war und die Pumprichtung alle 60 Sekunden umgekehrt wurde.

In Fig. 4 ist die dabei in dem Reaktionsraum beobachtete Konzentrationsänderung dargestellt. Aufgetragen ist auf der Ordinate der natürliche Logarithmus der Ausgangskonzentration dividiert durch die aktuelle Konzentration In C₀/C gegen die Zeit in Minuten. Man erkennt einen linearen Verlauf. Dies entspricht einer exponentiellen Abnahme der Konzentration, wie sie für einen gebräuchlichen Durchflußreaktor mit einer kontinuierlich durchströmten Membran charakteristisch ist. Diese Übereinstimmung der Antwortfunktion zeigt, daß die Versorgungsverhältnisse für das produzierende System bei der vorliegenden Erfindung ebenso gut sind wie bei dem Durchflußreaktor. Die Volumenverhältnisse, der Bedarf an produzierendem System (insbesondere mRNA) und die Konzentration des Produkts im Eluat sind jedoch wesentlich besser.

Durch die Erfindung wird somit insgesamt eine sehr effektive Durchführung biochemischer Reaktionen mit verhältnismäßig geringem Aufwand möglich. Daneben werden im einzelnen folgende Vorteile erreicht:
- Die Relation des Volumens des Reaktionsraums zu dem Volumen des Versorgungsraums ("Phasenverhältnis) läßt sich in weiten Grenzen wählen. Insbesondere kann mit einem geringen Reaktionsraumvolumen gearbeitet werden. In Verbindung mit der Modulation des Pumpvorgangs wird die Bildung von Sekundärfilterschichten und die daraus resultierende Verarmung der biologisch aktiven Makromoleküle in dem Reaktionsraum vermieden.
- Die durch die Erfindung mögliche flache Form des Bioreaktors erleichtert das Befüllen des Reaktionsraums und ermöglicht auf einfache Weise eine effektive Entlüftung.
- Dadurch, daß der Versorgungsraum glattflächig in seiner Längsrichtung durchströmt wird und insbesondere die Kapillaren vollständig durchströmt werden, werden Ablagerungen vermieden. Zugleich wird eine vollständige Überführung des gesamten Reaktionsprodukts zur Weiterverarbeitung gewährleistet, ohne daß Spülvorgänge mit resultierenden Ausdünnungseffekten erforderlich sind.
- Der Modulkörper kann aus zwei baugleichen Einheiten hergestellt werden, in denen die Kapillaren flach angeordnet sind. Die durch den Kapillarinnenraum gebildeten Teilräume 4 und 5 können sehr einfach mit den erforderlichen Anschlüssen für die Ver- und Entsorgung verbunden werden.

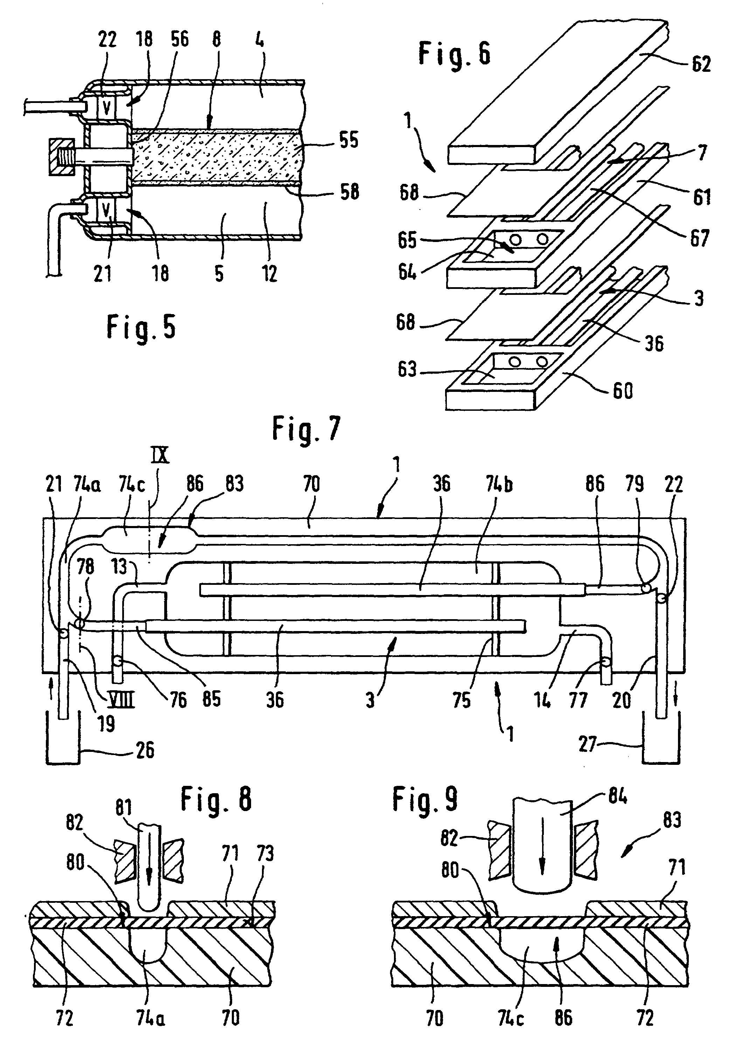

Bei der in Figur 5 dargestellten Ausführungsform ist der Reaktionsraum 3 mit einem porösen Festkörper 55 ausgefüllt, in dessen Poren sich die Komponenten des produzierenden Systems befinden. Der poröse Festkörper kann beispielsweise eine Glasfritte sein oder aus einem porösen keramischen Material bestehen.

Das produzierende System kann dabei ebenso wie bei Figur 1 mittels der Anschlußöffnungen 13 und 14 in den porösen Festkörper 55 über dessen Stirnflächen 56 eingefüllt werden. Vorzugsweise ist jedoch mindestens ein Teil der Komponenten des produzierenden Systems in dem porösen Festkörper 55 vorgepackt enthalten, d.h. diese Komponenten werden bereits bei der Herstellung des Bioreaktors 1 in die Poren des Festkörpers 55 eingebracht und der Anwender wird mit einem Bioreaktor beliefert, der einen Großteil der Bestandteile des produzierenden Systems bereits enthält. Dabei ist die Lagerung in einem porösen Festkörper vorteilhaft. Es ist grundsätzlich jedoch auch möglich, einen Bioreaktor gemäß den anderen Ausführungsformen der Erfindung in der Form herzustellen, daß der Reaktionsraum 3 einen Teil der Komponenten des produzierenden Systems vorgepackt (beispielsweise in Form einer gefriergetrockneten Schicht auf dessen Wänden oder als Tablette) enthält.

Bei der Ausführungsform mit einem porösen Festkörper gemäß Figur 5 ist vorzugsweise die Versorgungsmembran 8 an dessen äußerer Hüllfläche lokalisiert. Beispielsweise kann eine feinporöse Membranschicht 58 als äußere Umhüllung auf den porösen Festkörpers 55 aufgedampft sein. In neuerer Zeit stehen auch Fertigungsmethoden zur Herstellung eines einstückigen porösen Festkörpers 55 zur Verfügung, dessen äußere Oberfläche wesentlich feinporiger als sein Inneres ist, so daß die feinporige äußere Oberfläche eine semipermeable Membranschicht 58 bildet.

Figur 6 verdeutlicht, in welcher Weise bei einem Kapillarreaktor ähnlich den Figuren 2 und 3 eine zusätzliche Erntemembran vorgesehen werden kann. Das Gehäuse des Bioreaktors 1 besteht in diesem Fall im wesentlichen aus drei Formkörpern 60,61 und 62, die jeweils einen Sammelraum aufweisen, wobei in der Figur nur die Sammelräume 63 und 64 der Formkörper 60 und 61 zu erkennen sind. Die Sammelräume stehen jeweils in gleicher Weise wie bei Figur 2 und 3 einerseits mit (in Fig. 6 nicht dargestellten) Auschlußöffnungen und andererseits mit den Innenräumen von Kapillaren in Verbindung, wobei die Kapillaren 36 in den äußeren Formkörpern 60 und 62 die Versorgungsmembran 8 bilden, während die Erntemembran 7 durch in dem mittleren Formkörper 61 vorgesehene Kapillaren 67 gebildet wird. Innerhalb des durch die Formkörper 60,61 und 62 gebildeten Bioreaktormoduls sind die Sammelräume 63 und 64 sowie der nicht dargestellte Sammelraum des Formkörpers 62 durch Dichtungen 68 voneinander getrennt. Der Sammelraum 64 bildet gemeinsam mit einer in Figur 6 nicht dargestellten Anschlußöffnungen einen für die Kapillaren 67 der Erntemembran 7 gemeinsamen Leitungsanschluß 65 zum Ableiten der Flüssigkeit bei der Ernte des Produktes. Die Kapillaren 36 und 67, die die Versorgungsmembranen 8 und die Erntemembran 7 bilden, verlaufen in einem gemeinsamen Reaktionsraum 3, der durch den Außenraum zwischen den Kapillaren 36 und der diese umgebenden Gehäusewand gebildet wird.

Die Figuren 7 bis 9 sollen konstruktive Problemlösungen verdeutlichen, die für einen Mikromembranreaktor gemäß der vorstehend erläuterten Erfindung besonders geeignet sind, darüber hinaus jedoch auch selbständige Bedeutung für andere Bioreaktoren sehr kleiner Bauweise haben.

Der in den Figuren 7 bis 9 dargestellte Bioreaktor 1 besteht im wesentlichen aus einem Profilformkörper 70 aus Kunststoff, einem diesen großflächig abdeckenden Deckelteil 71 und einer zwischen dem Profilformkörper 70 und dem Deckelteil 71 angeordneten dünnen elastischen Folie 72. Die Folie 72 sollte aus einem hochelastischen inerten Material, wie beispielsweise Silikongummi, bestehen.

In der Deckfläche 73 des quaderförmigen Profilformkörpers 70 befinden sich insgesamt mit 74 bezeichnete Ausnehmungen. Die in dem Bioreaktor erforderlichen Leitungen werden durch schmale, nutförmige Ausnehmungen 74a gebildet, die eine Querschnittsfläche von beispielsweise 0,5 bis 1 mm² haben können. Der Reaktionsraum 3 wird von einer größeren trogförmigen Ausnehmung 74b gebildet, in der Hohlfasern (Kapillaren) 36 verlaufen, die mittels Haltestegen 75 fixiert sind.

Ebenso wie der Bioreaktor gemäß Figur 1 weist der Bioreaktor gemäß Figur 7 Ventile auf, um die Leitungen selektiv zu verschließen. Dargestellt sind Ventile 21 und 22 zum Verschließen der Versorgungsleitung 19 bzw. der Entsorgungsleitung 20 und Ventile 76,77, die die Anschlußöffnungen 13 und 14 des Reaktionsraumes 3 verschließen. In den Leitungsanschlüssen der Kapillaren 36 sind jeweils zusätzliche Ventile 78,79 vorhanden, die die nachfolgend erläuterte Pumpfunktion ermöglichen.

Die Funktion der Ventile 21,22 und 76 bis 79 wird anhand von Figur 8 deutlich. Die Folie 72 ist zwischen dem Deckelteil 71 und dem Profilformkörper 70 derartig eingespannt, daß sie die Randeinfassung 80 der Nut 74a abdichtend überdeckt. Über der Folie ist ein Ventilbetätigungsstift 81 in einer Führung 82 derartig vertikal beweglich, daß er in einer Verschlußposition des Ventils die Folie in die Nut drückt und in der dargestellten Offenposition die Folie freigibt, so daß sie die Nut überspannt. Die Betätigung des Stiftes 81 kann in bekannter Weise beispielsweise elektromagnetisch oder piezoelektrisch erfolgen.

Bei dem in Figur 7 dargestellten Bioreaktor 1 ist eine weitere Ausnehmung 74c vorgesehen, die Bestandteil einer Miniaturpumpe 83 ist. Die Ausnehmung 74c wird von der ihre Randeinfassung 80 abdichtend überdeckenden elastischen Folie 72 nach oben verschlossen. Die mit der Ausnehmung 74c verbundenen Leitungen sind jeweils durch Ventile 20,21,78,79 verschließbar. Der Hohlraum der Ausnehmung 74c bildet zusammen mit den an ihn angeschlossenen Leitungsabschnitten bis zu den Ventilen 20,21,78,79 ein Pumpraumvolumen 86.

Über der Folie befindet sich, wiederum durch eine Führung 82 geführt, ein Pumpenbetätigungsstift 84, der in einer ersten Position die Folie in das Pumpraumvolumen 74c drückt, so daß er dessen Volumen vermindert und in einer zweiten Position die Folie 72 freigibt, so daß sie das Pumpraumvolumen 74c überspannt.

Im dargestellten Fall dient die Pumpe 83 sowohl zum Ansaugen und Ablassen der Versorgungsflüssigkeit über die Versorgungsleitung 19 und die Entsorgungsleitung 20, die durch die Ventile 21 und 22 verschließbar sind, als auch zum Umpumpen während der Biosynthese durch die Anschlußleitungen 85 und 86 der Kapillaren 36. Die Funktionsweise wird beispielhaft anhand des Ansaugens von Versorgungsflüssigkeit aus dem Behälter 26 erläutert.

Zunächst wird das Ventil 21 geöffnet und der Pumpenbetätigungsstift 84 nach unten bewegt, um das Pumpraumvolumen 86 zu verkleinern. Danach wird die Ansaugöffnung der Versorgungsleitung 19 in die in dem Behälter 26 befindliche Versorgungsflüssigkeit eingetaucht und durch Anheben des Pumpenbetätigungsstiftes 84 mit resultierender Vergrößerung des Pumpraumvolumens 86 angesaugt. Das Ventil 21 wird geschlossen und das Ventil 22 geöffnet. Durch Absenken des Pumpenbetätigungsstiftes 84 wird die Flüssigkeit in Richtung auf das Ventil 22 gedrückt. Nach Schließen des Ventils 22 und Öffnen des Ventils 21 erfolgt mit dem nächsten Anheben des Pumpenbetätigungsstiftes 84 ein erneuter Ansaugvorgang. Durch zyklische Wiederholung dieses Vorganges kann jede gewünschte Flüssigkeitsmenge durch die Leitung gepumpt werden, in der sich die Pumpe 83 befindet. Das Umpumpen durch die Kapillaren 36 erfolgt analog unter Benutzung der Ventile 78 und 79, wobei die Ventile 21 und 22 geschlossen sind.

Insgesamt ermöglichen somit die anhand der Figuren 7 bis 9 erläuterten Konstruktionsmerkmale eine einfache und zuverlässige Betriebsweise eines Bioreaktors mit sehr kleiner Bauweise.

## Patentansprüche

1. Verfahren zur zellfreien Polypeptid-Biosynthese, mittels eines Bioreaktors, der der innerhalb eines Gehäuses (2)
A) in einem Reaktionsraum (3) ein produzierendes System mit mRNA oder einer Quelle für mRNA enthält und
B) in einem Versorgungsraum (6) einen von dem Reaktionsraum (3) durch eine semipermeable Membran (8) getrennten, als Versorgungsmedium für das produzierende System wirkende Flüssigkeit mit ATP. GTP und Aminosäuren enthält, wobei der Versorgungsraum (6) innerhalb des Bioreaktors in einen ersten und einen zweiten Teilraum (4, 5) geteilt ist, die
a) jeweils über eine semipermeable Versorgungsmembran (8) mit dem Reaktionsraum (3) in Verbindung stehen und
b) über eine Verbindungsleitung (15) miteinander verbunden sind,
bei welchem während der biochemischen Reaktion die Flüssigkeit im Kreislauf von dem ersten Teilraum (4) des Versorgungsraums (6) durch den Reaktionsraum (3) in den zweiten Teilraum (5) des Versorgungsraums (6) umgepumpt wird, wobei simultan
i) Flüssigkeit aus dem ersten Teilraum (4) in den Reaktionsraum (3) strömt,
ii) Flüssigkeit von dem Reaktionsraum (3) in den zweiten Teilraum (5) strömt und
iii) Flüssigkeit aus dem zweiten Teilraum (5) über die Verbindungsleitung in den ersten Teilraum (4) zurückströmt.

2. Verfahren nach Anspruch 1, bei welchem das Flüssigkeitsvolumen in dem Reaktionsraum (3) und in den Teilräumen (4,5) des Versorgungsraums (6) während des Umpumpens unverändert bleibt, so daß zu jedem Zeitpunkt der Volumenstrom der Flüssigkeit in den Reaktionsraum (3) hinein und der Volumenstrom der Flüssigkeit aus dem Reaktionsraum (3) heraus übereinstimmt.

3. Verfahren nach einem der vorhergehenden Ansprüche, bei welchem ein von dem produzierenden System erzeugtes Produkt aus der Versorgungsflüssigkeit gewonnen wird.

4. Verfahren nach einem der vorhergehenden Ansprüche, bei welchem zur Gewinnung eines von dem produzierenden System erzeugten Produktes Flüssigkeit aus dem Reaktionsraum über eine semipermeable Erntemembran (7) abgeleitet wird.

5. Verfahren nach einem der vorhergehenden Ansprüche, bei welchem während der biochemischen Reaktion die Pumprichtung und damit die Strömungsrichtung der Flüssigkeit, vorzugsweise mehrfach, umgekehrt wird.

6. verfahren nach Anspruch 5, bei welchem das in eine Richtung bis zur Umkehrung der Pumprichtung gepumpte Volumen mindestens so groß wie das Volumen des Reaktionsraums ist.

7. Vorrichtung zur Durchführung des Verfahrens nach einem der vorhergehenden Ansprüche
mit einem Bioreaktor, der innerhalb eines Gehäuses (2) einen ein produzierendes System mit mRNA enthaltenden Reaktionsraum (3) und einen von dem Reaktionsraum (3) durch eine semipermeable Membran (8) getrennten, eine als Versorgungsmedium für das produzierende System wirkende Flüssigkeit enthaltenen Versorgungsraum (6) aufweist, wobei der Versorgungsraum (6) innerhalb des Bioreaktors in einen ersten und einen zweiten Teilraum (4,5) geteilt ist, die jeweils über eine semipermeable Versorgungsmembran (8) mit dem Reaktionsraum in Verbindung stehen und über eine Verbindungsleitung (15) miteinander verbunden sind,
bei welcher
jeder der Teilräume (4,5) des Versorgungsraums eine Anschlußöffnung (17) für die Verbindungsleitung (15) aufweist und in der Verbindungsleitung (15) eine Pumpe (16) zum Umpumpen der Flüssigkeit angeordnet ist, und
die Versorgungsmembran (8) asymmetrisch mit einer einer ersten Oberfläche (9) der Membran (8) zugewandten Filtrationsschicht (10) und einer einer zweiten Oberfläche (11) der Membran (8) zugewandten Tragschicht (12) ausgebildet ist und die erste Oberfläche (9) zu dem Reaktionsraum (3) orientiert ist.

8. Vorrichtung nach Anspruch 7, bei welcher die Verbindungsleitung (15) und die Pumpe (16) in dem Gehäuse (2) des Bioreaktors (1) integriert sind.

9. Vorrichtung nach Anspruch 7, bei welcher der erste und der zweite Teilraum (4,5) des Versorgungsraums (6) an der Anschlußöffnung (17) für die Verbindungsleitung (15) jeweils eine Anschlußkupplung (47) zum Anschließen einer außerhalb des Gehäuses (2) des Bioreaktors (1) verlaufenden Verbindungsleitung (15) aufweist.

10. Vorrichtung nach einem der Ansprüche 7 bis 9, bei welcher die Versorgungsmembran (8) Membran-Kapillaren (36) aufweist und der Reaktionsraum (3) durch den Außenraum zwischen der Wand (49) des Gehäuses (2) des Bioreaktors (1) und den Kapillaren (36) gebildet wird.

11. Vorrichtung nach Anspruch 10, bei welcher
die Innenräume eines ersten Satzes (34) der Kapillaren (36) an beiden Enden der Kapillaren (36) jeweils einen für die Kapillaren des Satzes gemeinsamen Leitungsanschluß (39a,43;40a,45) aufweisen und den ersten Teilraum (4) des Versorgungsraums (6) bilden,
die Innenräume eines zweiten Satzes (35) der Kapillaren (36) an beiden Enden der Kapillaren (36) jeweils einen für alle Kapillaren (36) des Satzes gemeinsamen Leitungsanschluß (39b,44;40b,46) aufweisen und den zweiten Teilraum (5) des Versorgungsraums (6) bilden und
die Kapillaren der beiden Sätze (34,35) im wesentlichen parallel zueinander innerhalb des Reaktionsraums (3) verlaufen.

12. Vorrichtung nach Anspruch 11, bei welcher sich der überwiegende Teil des Volumens des Reaktionsraums (3) zwischen den beiden Sätzen (34,35) der Kapillaren befindet.

13. Vorrichtung nach einem der vorhergehenden Ansprüche, bei welcher der Reaktionsraum über eine semipermeable Erntemembran (7), deren Durchlaßgrenze größer als die der Versorgungsmembran ist, mit einer Leitung (29) in Verbindung steht, durch die zum Ernten eines von dem produzierenden System erzeugten Produktes Flüssigkeit aus dem Reaktionsraum (3) abgeleitet werden kann.

14. Vorrichtung nach Anspruch 13, bei welcher die Erntemembran (7) einen Satz von Membran-Kapillaren (67) aufweist, und die Innenräume der Kapillaren (67) an beiden Enden jeweils einen für die Kapillaren des Satzes gemeinsamen Leitungsanschluß (65) zum Ableiten der Flüssigkeit aufweisen.

15. Vorrichtung nach einem der vorhergehenden Ansprüche, bei welcher der Reaktionsraum einen porösen Festkörper (55) zur Aufnahme von mindestens einem Teil der Komponenten des produzierenden Systems enthält.

16. Vorrichtung nach einem der vorhergehenden Ansprüche, bei welcher die Versorgungsmembran (8) die äußere Hüllfläche des porösen Festkörpers (55) bildet.

17. Vorrichtung nach einem der vorhergehenden Ansprüche, bei welcher der Bioreaktor zur Bildung eines Miniaturventils eine von einer Randeinfassung (80) begrenzte Nut (74a) und eine die Randeinfassung (80) abdichtend überdeckende elastische Folie (72) aufweist, wobei über der Folie (72) ein Ventilbetätigungsstift (81) derartig beweglich ist, daß er in einer Verschlußposition des Ventils die Folie (72) in die Nut drückt und in einer Offenposition die Folie (72) freigibt, so daß sie die Nut (74a) überspannt.

18. Vorrichtung nach einem der vorhergehenden Ansprüche, bei welcher der Bioreaktor zur Bildung einer Miniaturpumpe (83) eine von einer Randeinfassung (80) begrenzte Ausnehmung (74c) und eine die Randeinfassung (8Q) abdichtend überdeckende elastische Folie (72) aufweist, durch die ein Pumpraumvolumen (86) begrenzt wird, das zwei jeweils mit einem Ventil verschließbare mit der Ausnehmung (74c) verbundene Leitungsabschnitte und den Innenraum der Ausnehmung (74c) umfaßt, und bei welcher über der Folie ein Pumpenbetätigungsstift derartig beweglich ist, daß er in einer ersten Position die Folie in die Ausnehmung (74c) drückt, so daß das Pumpraumvolumen vermindert wird und in einer zweiten Position die Folie freigibt, so daß sie die Ausnehmung (74c) überspannt.

19. Vorrichtung nach einem der vorhergehenden Ansprüche, bei welcher die Durchlaßgrenze der semipermeablen Versorgungsmembran (8) über 1.000 Dalton bis 5.000 Dalton liegt.

20. Vorrichtung nach einem der vorhergehenden Ansprüche, bei welcher die Durchlaßgrenze der semipermeablen Versorgungsmembran (8) unter 100.000 Dalton, vorzugsweise unter 50.000 Dalton liegt.

## Claims

1. Method for cell-free polypeptide biosynthesis, by means of a bioreactor comprising, within a housing (2),
A) a reaction chamber (3) containing a producing system with mRNA or a source for mRNA, and
B) a supply chamber (6) containing a liquid serving as a supply medium for the producing system and containing ATP, GTP and amino acids, which liquid is separated from the reaction chamber (3) by a semi-permeable membrane (8), wherein the supply chamber (6) within the bioreactor is subdivided into a first partial chamber (4) and a second partial chamber (5) which
a) are each connected to the reaction chamber (3) via a semi-permeable supply membrane (8) and
b) are connected to each other via a connection conduit (15),
wherein liquid is circulated during the biochemical reaction from the first partial chamber (4) of the supply chamber (6) through the reaction chamber (3) into the second partial chamber (5) of the supply chamber (6), the liquid simultaneously
i) passing from the first partial chamber (4) into the reaction chamber (3),
ii) passing from the reaction chamber (3) into the second partial chamber (5), and
iii) flowing out of the second partial chamber (5) via the connection conduit back into the first partial chamber (4).

2. Method according to claim 1, wherein the liquid volume in the reaction chamber (3) and in the partial chambers (4, 5) of the supply chamber (6) remains constant during circulation so that the volume flow of liquid into the reaction chamber (3) is always equal to the volume flow of liquid out of the reaction chamber (3).

3. Method according to any one of the preceding claims, wherein a product produced by the producing system is extracted from the supply liquid.

4. Method according to any one of the preceding claims, wherein liquid is removed from the reaction chamber via a semi-permeable harvesting membrane (7) to extract a product produced by the producing system.

5. Method according to any one of the preceding claims, wherein during the biochemical reaction, the pumping direction and thereby the flow direction of the liquid is reversed, preferably a plurality of times.

6. Method according to claim 5, wherein the volume pumped in one direction up to reversal of the pumping direction is at least as large as the volume of the reaction chamber.

7. Apparatus for carrying out the method according to any one of the preceding claims,
comprising a bioreactor, having, within a housing (2), a reaction chamber (3) containing a producing system and a supply chamber (6) containing a liquid with mRNA serving as a supply medium for the producing system and separated from the reaction chamber (3) by means of a semi-permeable membrane (8), wherein the supply chamber (6) within the bioreactor is subdivided into a first partial chamber (4) and a second partial chamber (5) which are each connected to the reaction chamber via a semi-permeable supply membrane (8) and which are connected to each other by means of a connection conduit (15), wherein
each of the partial chambers (4, 5) of the supply chamber has a connecting opening (17) for the connection conduit (15) and a pump (16) is disposed in the connection conduit (15) for circulating the liquid, and
the supply membrane (8) is formed in an asymmetrical fashion with a filtration layer (10) facing a first surface (9) of the membrane (8) and a support layer (12) facing a second surface (11) of the membrane (8), with the first surface (9) being directed towards the reaction chamber (3).

8. Apparatus according to claim 7, wherein the connection conduit (15) and the pump (16) are integrated in the housing (2) of the bioreactor (1).

9. Apparatus according to claim 7, wherein the first and the second partial chambers (4, 5) of the supply chamber (6) each have, at the connecting opening (17) for the connection conduit (15), a connection coupling (47) adapted for connection to a connection conduit (15) which extends outside of the housing (2) of the bioreactor (1).

10. Apparatus according to any one of claims 7 to 9, wherein the supply membrane (8) comprises capillaries (36) and the reaction chamber (3) is formed by the outer volume between the wall (49) of the housing (2) of the bioreactor (1) and the capillaries (36).

11. Apparatus according to claim 10, wherein
the inner spaces of a first set (34) of capillaries (36) each have a common conduit connection (39a, 43; 40a, 45) for the capillaries of the set at both ends of the capillaries (36) and form the first partial chamber (4) of the supply chamber (6),
the inner spaces of a second set (35) of the capillaries (36) each have a common conduit connection (39b, 44; 40b, 46) for all capillaries (36) of the set on both ends of the capillaries (36) and form the second partial chamber (5) of the supply chamber (6), and
the capillaries of both sets (34, 35) are located substantially parallel to each other within the reaction chamber (3).

12. Apparatus according to claim 11, wherein the major part of the volume of the reaction chamber (3) is located between the two sets (34, 35) of capillaries.

13. Apparatus according to any one of the preceding claims, wherein the reaction chamber is connected to a conduit (29) via a semi-permeable harvest membrane (7) having a MW-cutoff which is larger than that of the supply membrane and by means of which liquid is removed from the reaction chamber (3) for harvesting a product produced by the producing system.

14. Apparatus according to claim 13, wherein the harvesting membrane (7) comprises a set of membrane capillaries (67) and the inner spaces of the capillaries (67) each have, at both ends, a common conduit connection (65) for the capillaries of the set to remove the liquid.

15. Apparatus according to any one of the preceding claims, wherein the reaction chamber comprises a porous body (55) for acceptance of at least part of the components of the producing system.

16. Apparatus according to any one of the preceding claims, wherein the supply membrane (8) forms the outer jacket of the porous body (55).

17. Apparatus according to any one of the preceding claims, wherein the bioreactor has, for formation of a miniature valve, a groove (74a) defined by an edge (80) and an elastic foil (72) sealingly covering the edge (80), wherein a valve operation pin (81) is movable above the foil (72) in such a manner that, in a closed position of the valve, it presses the foil (72) into the groove and, in an opened position, releases the foil (72) so that it is stretched over the groove (74a).

18. Apparatus according to any one of the preceding claims, wherein the bioreactor has, for formation of a miniature pump (83), a recess (74c) defined by an edge (80) and an elastic foil (72) sealingly covering the edge (80), thereby defining a pump chamber volume (86) which includes two conduit sections, each of which can be closed by a valve, which are connected to the recess (74c) and the inner volume of the recess (74c), and wherein a pump operation pin is movable above the foil in such a manner that, in a first position, it pushes the foil into the recess (74c) to reduce the pumping chamber volume and, in a second position, releases the foil so that it is stretched over the recess (74c).

19. Apparatus according to any one of the preceding claims, wherein the MW-cutoff of the semi-permeable supply membrane (8) is more than 1,000 to 5,000 Dalton.

20. Apparatus according to any one of the preceding claims, wherein the MW-cutoff of the semi-permeable supply membrane (8) is less than 100,000 Dalton, preferably less than 50,000 Dalton.

## Revendications

1. Procédé pour réaliser une biosynthèse de polypeptides sans cellules au moyen d'un bioréacteur comprenant à l'intérieur d'une cuve (2),
A) dans un espace de réaction (3), un système de production contenant du mRNA ou une source de mRNA et
B) dans un espace d'alimentation (6) séparé de l'espace de réaction (3) par une membrane (8) semi-perméable, un liquide contenant de l'ATP, GTP et des aminoacides qui joue le rôle de milieu d'alimentation du système de production, l'espace d'alimentation (6) à l'intérieur du bioréacteur étant divisé en un premier et un deuxième compartiment (4, 5) qui,
a) chacun par l'intermédiaire d'une membrane d'alimentation (8) semi-perméable, communiquent avec l'espace de réaction (3) et
b) sont reliés entre eux par l'intermédiaire d'une conduite (15),
dans lequel le liquide contenu dans le circuit est pompé pendant la réaction biochimique du premier compartiment (4) de l'espace d'alimentation (6) à travers l'espace de réaction (3) dans le deuxième compartiment (5) de l'espace d'alimentation (6) et dans lequel, simultanément,
i) du liquide s'écoule du premier compartiment (4) dans l'espace de réaction (3),
ii) du liquide s'écoule de l'espace de réaction (3) dans le deuxième compartiment (5) et
iii) du liquide s'écoule du deuxième compartiment (5) via la conduite dans le premier compartiment (4).

2. Procédé selon la revendication 1, dans lequel le volume de liquide dans l'espace de réaction (3) et dans les compartiments (4, 5) de l'espace d'alimentation (6) reste inchangé pendant le pompage de manière à ce que, à tout moment, le débit volumique du liquide entrant dans l'espace de réaction (3) et le débit volumique du liquide sortant de l'espace de réaction (3) correspondent.

3. Procédé selon l'une des revendications précédentes, dans lequel on obtient un produit fabriqué par le système de production à partir du liquide d'alimentation.

4. Procédé selon l'une des revendications précédentes, dans lequel, afin d'obtenir un produit fabriqué par le système de production, on évacue du liquide de l'espace de réaction par l'intermédiaire d'une membrane de récolte (7) semi-perméable.

5. Procédé selon l'une des revendications précédentes, dans lequel le sens de pompage et donc le sens d'écoulement du liquide est inversé pendant la réaction biochimique, de préférence à plusieurs reprises.

6. Procédé selon la revendication 5, dans lequel le volume pompé dans un sens jusqu'à l'inversion du sens de pompage est au moins aussi grand que le volume de l'espace de réaction.

7. Appareil pour la mise en oeuvre du procédé selon l'une des revendications précédentes,
avec un bioréacteur, lequel comprend à l'intérieur d'une cuve (2) un espace de réaction (3) intégrant un système de production contenant du mRNA et un espace d'alimentation (6) séparé de l'espace de réaction (3) par une membrane (8) semi-perméable et contenant un liquide jouant le rôle de milieu d'alimentation du système de production, l'espace d'alimentation (6) à l'intérieur du bioréacteur étant divisé en un premier et un deuxième compartiment (4, 5) qui, chacun via une membrane d'alimentation (8) semi-perméable, communiquent avec l'espace de réaction et sont reliés entre eux par l'intermédiaire d'une conduite (15),
dans lequel chacun des compartiments (4, 5) de l'espace d'alimentation présente un orifice de raccordement (17) pour la conduite (15), la conduite (15) intégrant une pompe (16) pour le pompage du liquide, et dans lequel la membrane d'alimentation (8) est de forme asymétrique avec une couche de filtration (10) placée en regard d'une première surface (9) de la membrane (8) et une couche de support (12) placée en regard d'une deuxième surface (11) de la membrane (8), la première surface (9) étant orientée vers l'espace de réaction (3).

8. Appareil selon la revendication 7, dans lequel la conduite (15) et la pompe (16) sont intégrés dans la cuve (2) du bioréacteur (1).

9. Appareil selon la revendication 7, dans lequel le premier et le deuxième compartiment (4, 5) de l'espace d'alimentation (6) présentent chacun au niveau de l'orifice de raccordement (17) pour la conduite (15) un accouplement (47) pour le raccordement d'une conduite (15) s'étendant à l'extérieur de la cuve (2) du bioréacteur (1).

10. Appareil selon l'une des revendications 7 à 9, dans lequel la membrane d'alimentation (8) présente des capillaires (36) de membrane, l'espace de réaction (3) étant formé par l'espace extérieur entre la paroi (49) de la cuve (2) du bioréacteur (1) et les capillaires (36).

11. Appareil selon la revendication 10, dans lequel
les espaces intérieurs d'un premier groupe (34) de capillaires (36) ont aux deux extrémités des capillaires (36) respectivement un raccordement (39a, 43 ; 40a, 45) commun aux capillaires du groupe et forment le premier compartiment (4) de l'espace d'alimentation (6),
les espaces intérieurs d'un deuxième groupe (35) de capillaires (36) ont aux deux extrémités des capillaires (36) respectivement un raccordement (39b, 44 ; 40b, 46) commun à tous les capillaires (36) du groupe et forment le deuxième compartiment (5) de l'espace d'alimentation (6) et les capillaires des deux groupes (34, 35) s'étendent de manière essentiellement parallèle les uns aux autres à l'intérieur de l'espace de réaction (3).

12. Appareil selon la revendication 11, dans lequel la majeure partie du volume de l'espace de réaction (3) se trouve entre les deux groupes (34, 35) de capillaires.

13. Appareil selon l'une des revendications précédentes, dans lequel l'espace de réaction communique par l'intermédiaire d'une membrane de récolte (7) semi-perméable, dont le seuil de perméabilité est supérieur à celui de la membrane d'alimentation, avec une conduite (29) permettant d'évacuer du liquide de l'espace de réaction (3) afin de récolter un produit fabriqué par le système de production.

14. Appareil selon la revendication 13, dans lequel la membrane de récolte (7) comprend un groupe de capillaires (67) de membrane et dans lequel les espaces intérieurs des capillaires (67) ont aux deux extrémités respectivement un raccordement (65) commun aux capillaires du groupe, destiné à évacuer le liquide.

15. Appareil selon l'une des revendications précédentes, dans lequel l'espace de réaction contient un solide poreux (55) destiné à absorber au moins une partie des composants du système de production.

16. Appareil selon l'une des revendications précédentes, dans lequel la membrane d'alimentation (8) forme la surface enveloppante du solide poreux (55).

17. Appareil selon l'une des revendications précédentes, dans lequel le bioréacteur, afin de former une vanne miniature, comprend une rainure (74a) délimitée par un bord périphérique (80) et une feuille (72) élastique recouvrant le bord périphérique (80) de manière étanche, une tige d'actionnement (81) de la vanne, placée au-dessus de la feuille (72), pouvant être mue de telle sorte qu'elle pousse la feuille (72) dans la rainure en position de fermeture de la vanne et libère la feuille (72) en position d'ouverture de manière à ce qu'elle recouvre la rainure (74a).

18. Appareil selon l'une des revendications précédentes, dans lequel le bioréacteur, afin de former une pompe miniature (83), comprend un creux (74c) délimité par un bord périphérique (80) et une feuille (72) élastique recouvrant le bord périphérique (80) de manière étanche, qui délimitent un volume de pompage (86) contenant deux tronçons de conduite obturables chacun par une vanne et communicant avec le creux (74c) et l'intérieur du creux (74c), et dans lequel une tige d'actionnement de la vanne, placée au-dessus de la feuille, peut être mue de telle sorte qu'elle pousse la feuille dans le creux (74c) dans une première position de manière à réduire le volume de pompage et libère la feuille dans une deuxième position de manière à ce qu'elle recouvre le creux (74c).

19. Appareil selon l'une des revendications précédentes, dans lequel le seuil de perméabilité de la membrane d'alimentation (8) semi-perméable est supérieur à 1000 Dalton jusqu'à 5000 Dalton.

20. Appareil selon l'une des revendications précédentes, dans lequel le seuil de perméabilité de la membrane d'alimentation (8) semi-perméable est inférieur à 100.000 Dalton, de préférence inférieur à 50.000 Dalton.
